# EUROPEAN PATENT SPECIFICATION

(11) **EP 4 355 282 B1**
(45) Date of publication and mention of the grant of the patent: **21.05.2025**
(21) Application number: 22735937.9
(22) Date of filing: 17.06.2022
(51) Int. Cl.: A61G 15/16, A61G 15/14

(54) **DENTAL CARE UNIT INSTRUMENT BRIDGE**
INSTRUMENTENBRÜCKE FÜR ZAHNPFLEGEEINHEIT
PONT D'INSTRUMENT D'UNITÉ DE SOINS DENTAIRES

(30) Priority: 17.06.2021 FI 20215714
(43) Date of publication of application: 24.04.2024
(73) Proprietor: Finndent OY, 00620 Helsinki (FI)
(72) Inventor: KIVELÄ, Tapani, 00620 Helsinki (FI); RANTANEN, Timo, 00620 Helsinki (FI)
(74) Representative: Laine IP Oy
(86) International application number: PCT/FI2022/050428
(87) International publication number: WO 2022/263726

(56) References cited:
- EP-A1- 3 309 794
- WO-A1-2006/012663
- DE-U1- 29 520 999
- JP-A- 2011 130 857
- KR-A- 20210 016 127
- US-A1- 2014 272 771
- US-B2- 10 434 024

## Description

### FIELD

Various example embodiments relate to a dental care unit instrument bridge, and in particular a dental care unit instrument bridge comprising a touch screen.

### BACKGROUND

Dental care unit instrument bridges are used for holding a plurality of dental instruments for examine, manipulate, treat, restore and remove teeth and surrounding oral structures. Generally the dental care unit instrument bridge comprises an instrument holder configured to hold at least one dental instrument, such as a dental scaler, a micromotor, a dental syringe, a curing light, etc. The dental care instrument bridge may further comprise one display for displaying information related to a dental treatment.

Publication EP3202387 discloses a dental care instrument bridge for holding a plurality of dental instruments. The dental instrument bridge comprises: instrument holder configured to hold at least a first dental instrument; and a display for displaying information related to a dental treatment and an instrument picked up. There is a need for improvements for dental care unit instrument bridges.

JP2011130857 discloses a dental care instrument bridge comprising a touchless screen that can be divided in two and a second touchless screen. The output of the user's selection is displayed on the same screen or part of the screen.

### SUMMARY

The invention is defined by the features of the independent claims. Some specific embodiments are defined in the dependent claims.

According to the invention, there is provided a dental care apparatus, comprising an instrument bridge comprising a first touch screen, a second touch screen, and an instrument holder, wherein the first touch screen and the second touch screen are independently configurable, and the apparatus comprises means for causing the apparatus to: receive a first input associated with a first operator accessing the apparatus, indicative at least of a role and/or dental treatment procedure for the first operator, receive a second input associated with a second operator accessing the apparatus, indicative at least of a role and/or dental treatment procedure for the second operator, define a first operator configuration on the basis of the received first input, wherein the first operator configuration is indicative of a first set of dental care unit control actions associated with the role and/or dental treatment procedure for the first operator, define a second operator configuration on the basis of the received second input, wherein the second operator configuration is indicative of a second set of dental care unit control actions associated with the role and/or dental treatment procedure for the second operator, generate a first display view for the first touch screen on the basis of the first operator configuration, wherein the first display view is specific for the role and/or dental treatment procedure for the first operator, and generate a second display view for the second touch screen on the basis of the second operator configuration, wherein the second display view is specific for the role and/or dental treatment procedure for the second operator. The means may comprise at least one processor and at least one memory including computer program code, wherein the at least one memory and computer program code are configured to, with the at least one processor, cause the performance of the apparatus.

According to a second aspect, there is provided a method for controlling a dental care apparatus comprising an instrument bridge comprising a first touch screen, a second touch screen, and an instrument holder section, wherein the first touch screen and the second touch screen are independently configurable, the method comprising: receiving a first input associated with a first operator accessing the apparatus, indicative at least of a role and/or dental treatment procedure for the first operator, receiving a second input associated with a second operator accessing the apparatus, indicative at least of a role and/or dental treatment procedure for the second operator, defining a first operator configuration on the basis of the received first input, wherein the first operator configuration is indicative of a first set of dental care unit control actions associated with the role and/or dental treatment procedure for the first operator, defining a second operator configuration on the basis of the received second input, wherein the second operator configuration is indicative of a second set of dental care unit control actions associated with the role and/or dental treatment procedure for the second operator, generating a first display view for the first touch screen on the basis of the first operator configuration, wherein the first display view is specific for the role and/or dental treatment procedure for the first operator, and generating a second display view for the second touch screen on the basis of the second operator configuration, wherein the second display view is specific for the role and/or dental treatment procedure for the second operator.

There is also provided an apparatus comprising at least one processor, at least one memory including computer program code, the at least one memory and the computer program code being configured to, with the at least one processor, cause the apparatus at least to carry out features in accordance with the method of the second aspect, or any embodiment thereof.

According to still further aspects, there are provided a computer program and a computer-readable medium, or a non-transitory computer-readable medium, comprising code configured, when executed in a data processing apparatus, to carry out features in accordance with the method of the second aspect, or an embodiment thereof.

### BRIEF DESCRIPTION OF THE DRAWINGS

Some example embodiments will now be described with reference to the accompanying drawings.
FIGURE 1a illustrates a dental care unit instrument bridge apparatus according to some embodiments,
FIGURE 1b illustrates an example dental care unit;
FIGURES 2 and 3 illustrate methods in accordance with at least some embodiments,
FIGURE 4 illustrates an example of an instrument bridge in accordance with an embodiment; and
FIGURE 5 illustrates a schematic diagram of a data processing apparatus according to some embodiments.

### EMBODIMENTS

A plurality of devices and settings of a dental care unit are used in accordance with performed treatment, such as tooth filling, tooth removal, bleaching, or root treatment. Dental professionals often work at different dental clinics or different dental rooms, often need to start working with new dental care units, and perform consecutively many dental treatments that often substantially differ from each other. Dental care units are permanently installed into treatment rooms which are often used by several dentists or dental hygienists. An object of at least some of the present embodiments is to facilitate use of dental care units and facilitate improvement for more safe, efficient and user-friendly dental treatment operations.

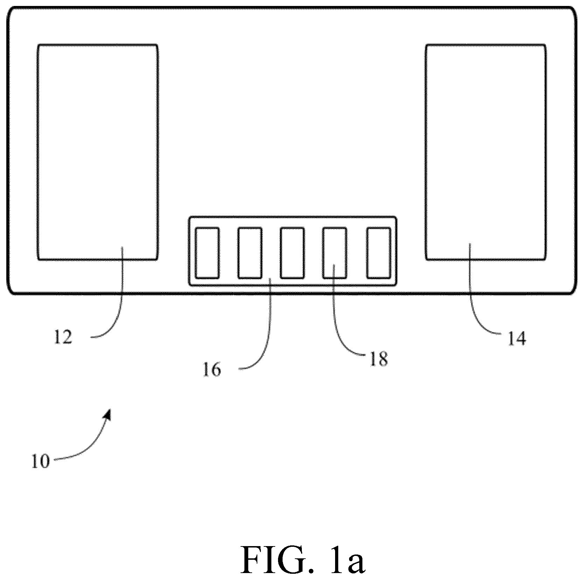

FIG. 1a illustrates a dental care unit instrument bridge unit or apparatus 10 in accordance with at least some embodiments. The dental care unit instrument bridge apparatus 10, comprises a first touch screen 12, a second touch screen 14, and an instrument holder 16 separate from the touch screens. The first touch screen 12 is positioned at a first end of the apparatus and the second touch screen 14 is positioned at a second end of the apparatus.

The instrument holder 16 may be arranged between the touch screens 12, 14 to receive a plurality of dental instruments at predetermined positions in front of the integral surface. The instrument holder may have holder portions 18 for receiving dental instruments.

A housing, in which the touch screens 12, 14 and the instrument holder 16 is arranged, may be connected to a movable arm (not shown) of a dental care unit for moving the apparatus. The movable arm is configured to allow the operator to move the apparatus at a desired position. The housing may further comprise a handle for moving the apparatus. An electric break can be included at the coupling of the bridge apparatus 10 to lock the bridge into the desired position. An integral surface may cover the first touch screen and the second touch screen. The instrument holder 16 is thus arranged to receive a plurality of dental instruments at predetermined positions in front of the integral surface.

The apparatus 10 may further comprise or be connected to a drive power and feed-system for providing drive power or feed to the dental instruments. The drive power or feed provided may comprise electricity, water, compressed air, vacuum, mechanical power, and/or light.

A set of dental care instruments may be attached to the instrument holder 16. There may be an instrument-specific holder portion 18 for each instrument of the set. Some examples of such instruments include a dental scaler, a micromotor, a turbine, a dental syringe, and a curing light.

A dental care unit, apparatus or system may comprise or be connected to the dental bridge apparatus 10. The apparatus 10 may be connected to a set of dental care related (sub-) units, such as ancillary devices or other devices or units of a dental care unit. Further devices connectable to the apparatus 10 may comprise instruments or instrument units used in dental treatment, as well as control devices. Examples of typical instruments in ancillary devices include light curers and syringes. A suction device for removing saliva and treatment byproducts from the mouth may also be included in the dental care unit and may in some embodiments be connected to the apparatus 10. A patient chair is usually attached to a dental care unit, in which case the patient chair can also be controlled via the control devices of the dental care unit. Other typically applied devices include an operation light and a flushingwater system, an x-ray device, a microscope, an intra-oral camera unit, and a monitor, for example.

The further devices connected to the apparatus 10 may comprise one or more actuators, referring generally to a control unit of the respective device, and/or one or more sensors, to which the apparatus 10 may be connected. For example, the apparatus 10 may be connected to one or more of a suction device actuator, to a flushing device or water supply control actuator, to a foot pedal sensor, a chair position actuator, etc.

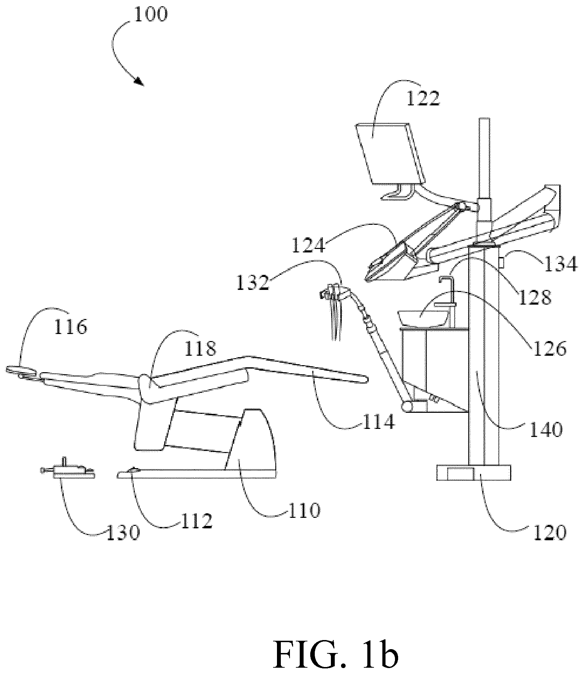

FIG. 1b illustrates, by way of example, a dental care unit 100. The dental care unit comprises a patient chair 110 and an accessory part 120. The dental care unit comprises a chair foot control, 112. Buttons of the chair foot control may cause pre-defined functions in the chair. For example, one button may cause lifting of the chair 110, and another button may cause lowering of the chair 110. The patient chair 110 comprises adjustable foot rest 114 and neck rest 116, and optionally arm rests 118. Position of the chair 110 may be locked by a swivel lock.

The dental care unit may comprise a foot pedal 130. Buttons of the foot 130 pedal may cause pre-defined functions in the dental care unit. Accessory part 120 of the unit comprises an instrument bridge 124, such as the dental bridge apparatus 10, connected to movable arm of the unit. Control panel may be part of the bridge 124. The control panel may comprise the touch screens 12, 14, in which specific operator views may be displayed, as further illustrated for apparatus 10 below. The accessory part 120 may comprise a further monitor 122 or a display, with larger screen size.

The dental care unit may comprise a cuspidor 126 and a faucet 128. Suction arm and holder 132 are connected to the pipes of the cuspidor. The dental care unit comprises one or more on/off buttons or switches. For example, the accessory part 120 may comprise a separate on/off button 134, and the chair 110 may comprise a separate on/off button. The button may be operated manually and/or electrically. For example, the dental care unit or at least the accessory part 120 may be locked by electrically setting the button 134 to off position. When the dental care unit is off, usage of the dental care unit is prevented.

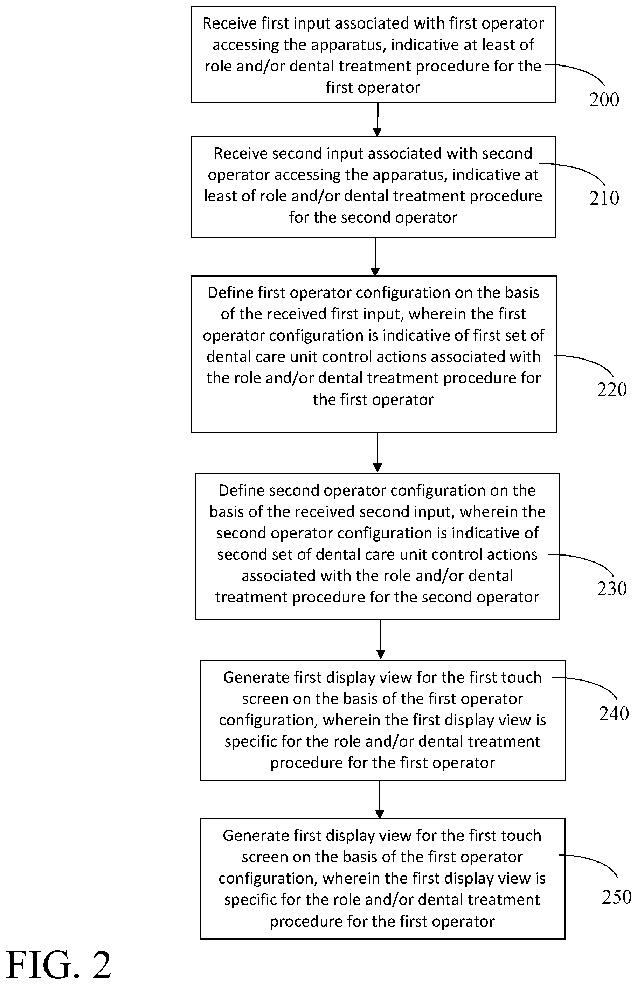

The dental care unit comprises a control unit, for example in the frame 140 or the bridge 124. The control unit comprises e.g. elements described in Fig. 6, and may be configured to perform the method of FIG 2. The control unit may be configured to control functions of the dental care unit. For example, the control unit may control turbines of the instruments, micromotors, syringe, suction, monitor 122, etc. For example, the control unit may control which information to display on the touch screens of the bridge 124. At least some of the chair, and other functional modules (instrument bridge, chair, monitor) of the dental care unit may comprise a control unit, and different control units may communicate with each other via a bus or buses, for example. The control unit of the chair may be configured to control the chair e.g. based on input received via the chair foot control 112. The foot pedal, chair foot control, and the chair are connected to the control unit residing in the frame 140, e.g. via wiring. The control unit may be configured to control any functional module of the dental care unit. Likewise, control panel of the dental care unit may be configured to control any functional module of the dental care unit.

The dental care unit comprises a unique identifier. The identifier may be, for example, a string of characters, such as numbers and/letters, a bar code, a QR code, or any suitable identifier. The dental care unit may comprise or may be coupled to one or more further units, such as detergent container(s). The containers may comprise different compounds for cleaning hoses of the dental care unit.

The first touch screen 12 and the second touch screen 14 may be independently configurable based on input information received by the instrument bridge apparatus 10. FIG. 2 illustrates a method for a dental care unit, and in particular operations of or related to an instrument bridge with (at least) two touch screens, such as the instrument bridge apparatus 10. The apparatus 10, or a control unit included in or connected to the apparatus 10, may be configured to perform the method of FIG. 2.

The method comprises receiving 200 a first input associated with a first operator accessing the apparatus, indicative at least of a role and/or dental treatment procedure for the first operator.

Block 210 comprises receiving a second input associated with a second operator accessing the apparatus, indicative at least of a role and/or dental treatment procedure for the second operator.

Block 220 comprises defining a first operator configuration on the basis of the received first input. The first operator configuration is indicative of a first set of dental care unit control actions associated with the role and/or dental treatment procedure for the first operator.

Block 230 comprises defining a second operator configuration on the basis of the received second input. The second operator configuration is indicative of a second set of dental care unit control actions associated with the role and/or dental treatment procedure for the second operator.

Block 240 comprises generating a first display view for the first touch screen on the basis of the first operator configuration. The first display view is specific for the role and/or dental treatment procedure for the first operator.

Block 250 comprises generating a second display view for the second touch screen on the basis of the second operator configuration, The second display view is specific for the role and/or dental treatment procedure for the second operator. Some further example embodiments are illustrated below. Alhough references are made to the example instrument bridge apparatus 10, it will be appreciated that at least some of the features may be performed by another unit of a dental care unit (apparatus), such as a control unit connected to the dental bridge.

The instrument bridge apparatus 10, or a control unit included in or connected to the apparatus 10, may be configured to control at least one set of dental care unit actuators on the basis of the first operator configuration and/or the second operator configuration. A first set of dental care unit actuators may be controlled based on the first set of dental care unit control actions. A second set of dental care unit actuators may be controlled based on the second set of dental care unit control actions. The first set of dental care unit control actions being associated with the first operator and the second set of dental care unit control actions being associated with the second operator facilitate use of the dental care unit by several operators. This kind of configuration takes into account the roles of the operators and/or the current dental treatment procedure.

For example, these dental care unit control actions may comprise one or more of controlling the patient chair to a position according to the identified dental treatment procedure or stage thereof, such as an entry position, a position for performing a specific treatment action (e.g. drilling), a position for spitting, a position for the patient to exit, a cleaning position, starting, turning of or adjusting a compressor, a water supply, or a suction, starting cleaning of suction hose(s), starting disinfection of input water lines, etc. Control signals indicative of the associated control actions can be transmitted to respective connected devices and instruments of the dental care unit in accordance with the first and/or second configuration.

Examples of treatment procedures are removal of a tooth, filling of a tooth, bleaching of teeth, root canal therapy, orthodontics, etc. The settings of the dental care unit may be different for different treatment procedures.

For example, a following treatment procedure scheduled for the dental care unit is root canal therapy. The instrument bridge may comprise five instruments, e.g. two different micromotors, a turbine, a syringe, and a calculus remover. Head piece of the micromotors may be changed in order to change the rotational speed of a drill. A turbine may be replaced with a micromotor, if the micromotor uses a specific, red head piece which multiplies the rotational speed of the motor. When starting the treatment (root canal therapy), a turbine or a micromotor with the red head piece is used in the opening phase. In the second phase, a micromotor with a blue head piece is used, i.e. the rotational speed of the drill and the motor is the same. Removal of tooth matter is finished with a micromotor with an endo head piece, which slows down the rotational speed of the drill below the rotational speed of the motor. If the root canal therapy is performed with micromotors with different head pieces, usage of the turbine, syringe and calculus remover may be prevented.

The input(s) of the method of FIG. 2 may comprise treatment identifier(s). The treatment identifier may identify a dental treatment procedure or procedure type, such as tooth filling, tooth removal, or root canal treatment. The operator configuration(s) may be defined 220, 230 by selecting a preconfigured treatment program associated with the respective treatment identifier. The preconfigured treatment program may comprise the respective (first/second) set of dental care unit control actions. For example, the treatment identifier is received from a patient or clinic information system.

The preconfigured treatment programs may be stored to a database or memory residing in or accessible by the dental care unit. The control unit may retrieve the treatment program associated with the respective treatment identifier and start to execute the program. During execution of program, the control unit may cause appropriate control signals to actuator(s) in accordance with dental care unit control actions associated with the current treatment procedure stage.

The input(s) may comprise role identifier(s). The input may indicate the role from a set of preconfigured roles, which may comprise at least some of: a dentist, a dental surgeon, a dental assistant, an oral hygienist, and a patient. It is to be noted that these are general or high-level examples of roles, and the roles may be defined in various other, in more specific ways. For example, there may be a set of dentist roles.

In an example, before beginning the actual dental treatment operations, a patient role may be input 210 as the second operator role, and the doctor may use the second touch screen for example, for displaying and explaining the treatment plan and showing X-ray pictures for the patient. After this, the role may be changed e.g. to dentist (or dental assistant) role and the display view adapted for the dental professional to start first stage of the identified dental treatment procedure.

Both displays can be set up independently and the content of the displays can be changed, e.g. depending on whether operators are left-handed or right-handed. Hence, either operator can choose its location freely while the content relevant and characteristic for each role can be selectively displayed in the respective touch screen. The preconfigured roles may be stored to a database or memory residing in or accessible by the dental care unit. Relevant role information may be retrieved by the control unit based on the received role identifier.

For example, the first input may indicate a role of a dental surgeon for the first operator. The first touch screen 12 may display, for example, patient-specific dental charting, a treatment history and/or surgery plan in the first display view assisting the surgeon for performing a specific dental surgery procedure identified in block 200. Further, the second input may indicate the role of a dental assistant for the second operator and the second display view be a dental surgery assistant or assistance view. The second touch screen 14 may thus display, for example, a dental surgery procedure view, information of allergies of the patient and charting, and/or display element assisting the dental assistant with preparing instruments at each stage of the procedure. Moreover, the touch screen(s) may display, for example, information about the dental instrument and information for the next stage of the dental treatment procedure. This improves the cooperation of the operators (dentist and assistant) especially when they are not familiar to each other.

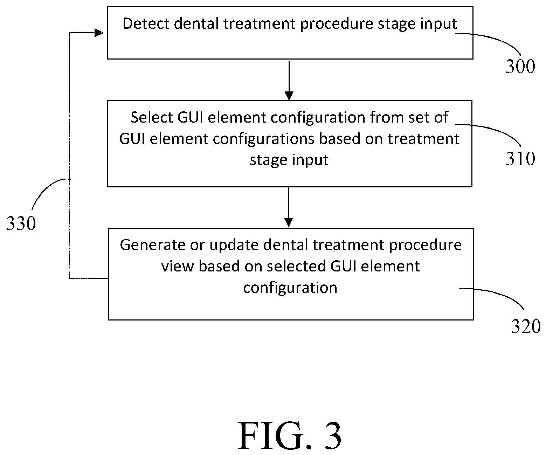

The first display view and/or the second display view may comprise a dental treatment procedure view comprising a treatment stage specific display element configuration. With reference to example method of FIG. 3, a dental treatment procedure stage input is detected 300. The input may be an input to change from one stage of the procedure to another. The input may be received via the touch screen 12 or 14 (or another input device of the dental care unit) or detected based on automatic stage transition of the control procedure being performed in the dental care unit. Triggering action for proceeding to a following stage of a treatment may be based on a user input, e.g. via the foot pedal, or based on detecting that the dentist has used a specific instrument and has put that back on the instrument tray.

A GUI element configuration is selected 310 among a set of treatment stage specific GUI element configurations for the dental treatment procedure based on the detected input. The dental treatment procedure view is generated or updated 320 based on the selected GUI element configuration. As further illustrated by transition 330, the procedure may continue to monitor for new stage (change) input and repeat steps 310 and 320 upon detecting state change. The method of FIG. 3 may be entered e.g. after block 230 or 250 and performed during execution of the dental treatment procedure.

According to some embodiments, the apparatus 10 may be configured to detect when a dental instrument is selected and/or detached from the instrument holder 16 by the first operator or the second operator, and response thereto control the first touch screen and/or the second touch screen to change the current view and display information relevant for the dental instrument. The apparatus 10 may identify a dental instrument detached from the instrument holder portion 18. The first touch screen and/or the second touch screen may be controlled to change the current view and display information associated with the identified dental instrument.

The apparatus 10 may be configured to detect selection and/or detachment from the holder by the operator by receiving a sensor or detector signal, or another type of signal indicative of the selection or detachment. Attachment or detachment of the dental instrument may be identified e.g. by sensor readings, such as proximity sensor readings or other suitable detector readings. Such signal may be received from the respective dental instrument, the instrument holder 16, the holder portion 18 and/or from another part of the dental care unit. In a simple example, the holder portion 18 for a specific instrument may comprise a switch indicative of state of the holder portion, i.e. if the instrument is attached to the portion or not. This enables the first touch screen and/or the second touch screen to display automatically information about the dental instrument, which facilitates dentist's work.

The detachment may be a further input, on the basis of which the first display view and/or the second display view is at least partially re-defined or updated. The first and/or second operator configuration may be re-defined on the basis of an identifier of the detached instrument. Detachment of the instrument may be a stage input and cause block 300 to be entered. Similarly, the apparatus 10 may identify attachment of a dental instrument to the instrument holder section, and control display view(s) based on the identifier of the attached instrument.

The first and/or second display view may be or comprise operator assistance display element(s), in accordance with the first operator configuration and/or the second operator configuration. The assistance display elements are updated in accordance with the treatment stage, and the dental care device and/or instrument being currently used.

For example, a display view indicative of required instruments, as well their further parameters and/or supplementary guidance information, for the dental treatment procedure being performed and their usage order during the procedure being performed may be displayed. For example, an image or a sequence of images of the respective instrument is displayed, with visual and/or audible guidance information according to the identified procedure, guiding to use the instrument appropriately at the current procedure stage. For example, the guidance information may comprise images to guide the operator to do certain actions, written instructions, and/or audible instructions. Depending on the current stage of the procedure, GUI element(s) may be prevented from being selected and used, e.g. by disabling inputs to them, thus further increasing safety in case of less-experienced operators. The above features further facilitate to have smoother co-operation for a less-experienced operator or for operators not knowing each other.

In another example, upon identifying the instrument detached from the holder, an associated assistance view is displayed. A further example is a dental assistant assistance view. During the sequential stages of the treatment procedure, a treatment procedure specific assistance view includes element(s) guiding the assistant at the currently executed stage. The assistance elements may be updated based on the procedure illustrated in connection with FIG. 3. For example, during a tooth filling procedure, at a stage preceding filling of the cavity, assistance element guides the assistant to prepare a filling instrument at the instrument holder with predefined filling material, so it will be ready for the dentist for the next stage. Assistance views may be similarly generated for other roles. This feature also enables new approach in training dentist and hygienists so that operators (teacher and student in this particular case) can vary their roles during the training process.

The display views may comprise instruction information specific to the role and/or treatment procedure. For example, a dentist can input assisting instructions beforehand so that this information may be selectively displayed in the assistant's display during the different phases of the dental treatment procedure.

Instructions may be generated for a treatment procedure such that it has been taken into account that during the treatment, both a dentist and an assistant are present in the treatment room. For example, the dentist may have generated specific instructions for his or her assistant, related to certain treatments. The dental care unit may recognize phases of the treatment based on which instruments have been used or are being used. Then, the instructions may comprise instructions to be presented to the assistant in response to dental care unit detecting a certain phase of the treatment. Instructions may be proactive in sense that the instructions concern the following phase of the detected phase, for example. This is beneficial if the dentist and assistant do not know each other beforehand, or they do not share a mutual language. The assistant might not know dentist's way of working. The instructions received with the configuration message and presented to the assistant, for example by displaying on the monitor, will guide the assistant through the treatment.

For example, let us consider the root canal therapy described above. When the dentist has used the micromotor with the red head piece in the opening phase, the dentist takes the micromotor with the blue head piece from the instrument bridge. Then, an instruction is presented to the assistant to change the red head piece to the endo head piece. When the dentist finishes using the micromotor with the blue head piece, the next instrument is ready for the dentist. The dentists typically use turbines and micromotors with different head pieces. Way of usage of different instruments may depend on the treatment in question and on personal preferences. Therefore, specific instructions presented to the assistant facilitate to improve patient safety by minimizing errors in changing the head pieces.

An operator-specific preference data set may be selected on the basis of an operator identifier detected or received based on the authentication of the operator. The display-specific view may be controlled based on the operator-specific view. For example, an operator may have stored a settings profile indicative of a preferred configuration (order, size, etc.) of UI elements and/or display settings (e.g. colour scheme), and the first display is configured based on this profile upon detecting the ID of the operator. Both displays of the instrument bridge may thus be adapted to personal preferences of the operator, in addition to the current role of the respective operator and/or the dental procedure being carried out.

A dental chair adjustment panel view may be displayed in the first touch screen and/or the second touch screen, and control a dental chair position actuator on the basis of an input to the dental chair adjustment panel view.

The apparatus 10 may be arranged to display a menu on the first touch screen 12 and the second touch screen 14. The menu may comprise icons. The icons may be related to for example, functions of the apparatus 10 and controlling of the dental instruments and a dental chair. Content of the menu, and the appearance and/or location of the icons may be set according to the first operator and/or the second operator preferences.

The first operator and the second operator can give input to the apparatus 10 by touching the first touch screen 12 and the second touch screen 14 with one or more fingers or a special stylus. Additionally, the apparatus 10 may further comprise input device(s), such as one or more of a keyboard, a mouse, a gesture input device, foot pedal, or other input device.

In some cases, same information can be displayed on the first touch screen 12 and the second touch screen 14 at the same time. For example, the treatment plan or X-ray pictures may be displayed to the first operator and the second operator at the same time. Moreover, a complete display view, a portion of displayed view, or some of the information in a display view may be controlled or duplicated from the first touch screen 12 to the second touch screen 14. This enables for example, the first operator and the second operator to exchange information during the treatment. Either one of the touch screens may be set as a master or ruling/prioritized input source. Thus, in case conflicting inputs are received via both touch screen, the input received via the master input source will be applied instead of the input from the other non-prioritized input source. For example, such setting may be performed based on role as indicated by the input(s) received 200, 210 and/or as based on the configuration of block 220, 230.

The apparatus 10 may be configured to display a dental patient chair adjustment, such as chair orientation, height of the chair and other ergonomics settings, on the first touch screen and/or the second touch screen for adjusting the dental chair. Moreover, the apparatus 10 may be configured to display chair preset settings for different treatments and/or patients on the first touch screen 12 and/or the second touch screen 14. This enables more safe and efficient treatment, when there is no need for selecting the settings separately for every treatment and/or patient. In a further example, the first display view 12 may comprise dental treatment stage or action related information and control elements, and the second display view 14 may comprise other/supplementary information and control elements, such as chair adjustment menu.

As already indicated, the apparatus 10 may be configured to display instrument settings and/or parameters on the first touch screen 12 and/or the second touch screen 14 depending on a dental instrument in use. Dental instrument parameters displayed by the first touch screen and/or the second touch screen may be for example, speed, torque, spray water on or off, spray air on or off, direction of rotation, time, intensity of a treatment light, air flow, and spray water on or off. At least some of these parameters may be included in the first and/or second operator configuration illustrated above.

The apparatus 10 may be configured to display preset definition for every treatment, such as instrument settings for a treatment plan, on the first touch screen 12 and/or the second touch screen 14 based on the first and/or second operator configuration. When the dental treatment comprise a plurality of stages, features illustrated in connection with FIG. 3 may be applied. For example, the first touch screen and/or the second touch screen may indicate to the first operator and/or the second operator which dental instrument should be used for the next step of the dental treatment.

The first and/or second display view may comprise a treatment imaging view, such as a camera view, a root canal treatment view or an X-ray image view, on the first touch screen and/or the second touch screen. In a further example, the view(s) may comprise GUI elements for comparing earlier stored camera images or videos, or X-ray images with currently imaged dental view.

The apparatus 10 may be configured to display time and date on the first touch screen 12 and/or the second touch screen 14. Moreover, a timer for treatments, such as time for prehardening of tooth filling, may be displayed.

The first and/or second configuration may comprise visualization parameters to generate the first display view and/or the second display view. The apparatus 10 may be configured to display treatment light settings for illuminating a treatment area on the first touch screen 12 and/or the second touch screen 14. The touch screen may be used to control for example, intensity of treatment light. This makes the first operator and/or second operator work easier when the area to be treated can be illuminated efficiently. For example, illumination may be varied during the treatment procedure by a non-active operator by his/her touch screen.

The apparatus 10 may be configured to control alarms on the first touch screen 14 and/or the second touch screen 16. The alarms may be silent. The first touch screen and/or the second touch screen may display the alarm, for example, when maintenance is needed.

In an example embodiment, the apparatus 10 may have an assistance call button on the first touch screen 12 and/or the second touch screen 14. The apparatus 10 may have door opening button on the first touch screen 12 and/or the second touch screen 14. Thus, the door may be easily opened for the patient or for the dental assistant when assistance is needed. The apparatus 10 may further have a touch screen or a touch lock button for switching on or off the first touch screen 12 and/or the second touch screen 14.

According to some embodiments, the apparatus 10 may be configured to display maintenance settings in the first display view and/or the second display view. The maintenance setting may be displayed for example, after predetermined incident or time, such as after each patient, after every working day or every week.

Another unit external to the apparatus 10, such as a network server, e.g. a cloud server, may provide a network service for controlling use of the dental care unit. The network service may comprise a database of users that are authorized to use the dental care unit. In addition, the network service may comprise a database comprising pre-configured settings for different dental care units. The settings may be tailored for different treatment procedures and/or for different users.

Communication from the network service to the dental care unit may be based on push messages. The communication between the devices may be based on, for example, the advanced message queuing protocol (AMQP) or message queuing telemetry transport (MQTT). Push messages may be encrypted and protected against interception, modification or forgery by using a cryptographic protocol, such as transport layer security (TLS).

The network service may authenticate the first operator and/or the second operator. This may be performed based on operator identification information received from a mobile device of the operator or another user device, such as a terminal device or a local server of a clinic IT system. For example, identification information may comprise a password, or information from a fingerprint or facial characteristics detection unit. Then, the network service may check and verify that the user is authorized to use the dental care unit.

The network service may also receive identification information of the dental care unit. The network service may define the first input and the second input based on, for example, the operator identification information, the dental care unit identification information and other treatment procedure related input, such as information on treatment procedure scheduled for the operator in a treatment scheduling system.

Before usage of the dental care unit, the operator, e.g. a dentist or a dentist's assistant, may thus need to sign in to the network service, which may then pair the operator and the dental care unit. The operator may thus get authorization to use the dental care unit via the network service. For pairing the operator and a particular dental care unit, the operator may transmit a message comprising at least the identifier of the dental care unit to the network service and the operator identification information, using the user device.

In some embodiments, the first input and the second input is received 200, 210 in at least one configuration or control message from the network service. The configuration message may be a push message and comprise a sequence of control parameters.

The push message, or another configuration message, may comprise the first operator configuration and/or the second operator configuration, or a sequence of control parameters for the first operator configuration and/or the second operator configuration. In this embodiment, the definition in block 220 and/or 230 may refer to or comprise receiving the configuration from the server or from a local memory, in which the configuration information has been earlier stored. All information may need to be loaded into to the unit before the treatment begins, since the dental care unit may not be able to receive any information during the treatment.

The network service may also have an access to a calendar or schedule of a clinic, wherein the dental care unit is located. The calendar comprises information on a schedule of the planned dental care procedures or treatment procedures related to a treatment room, where the dental care unit is located in. In addition, the calendar may comprise patient specific data. Based on the calendar, user id (operator and/or patient), and/or the identifier of the dental care unit, the network service may generate a configuration message to be sent to the dental care unit. The network service may have a pre-built database on different configurations or settings at least for different operator roles and for different treatment procedures. Settings of the dental care unit may be selected from the database for a specific treatment procedure and/or for the identified user, and a configuration message may be generated based on the selected settings. The configuration message is transmitted to the dental care unit. The configuration message may comprise information related to the particular dental care unit, role specific settings, and/or treatment specific settings and/or patient specific settings of the dental care unit. The configuration message may also include information that is provided for the network service by a third party. For example, the message may include information that is critical for treatment, patient, dentist and/or assistant but does not necessarily have a direct effect on dental care unit. This enables to improve patient safety and communication without disturbing the treatments while information will be received during the changeovers of treatment and/or patient. The configuration message may comprise assistance information and/or instructions to be displayed on the first display and/or the second display, such as the information illustrated above.

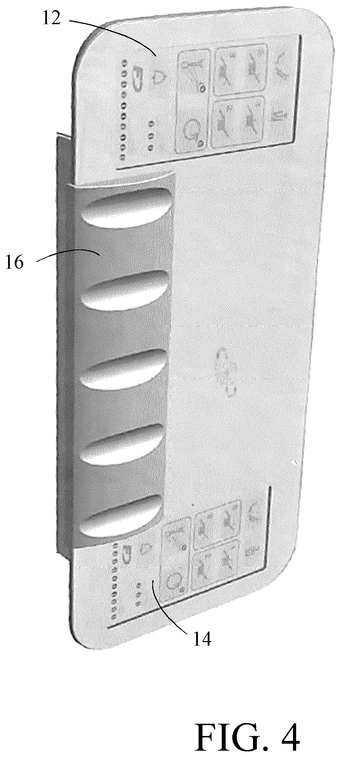

According to some embodiments, an integral surface covers at least the first touch screen 12 and the second touch screen 14. A one-piece transparent surface portion may cover or comprise at least the first touch screen and the second touch screen. The instrument holder is attached to and/or positioned on top of the surface portion. The instrument holder is arranged to receive a plurality of dental instruments at predetermined positions in front of the integral surface. FIG. 4 illustrates an example of such instrument bridge.

The integral surface may cover also the instrument holder 16. The integral surface facilitates sanitation of the dental care unit instrument bridge apparatus, because there are no or less grooves to which the dirt could catch.

The surface or surface portion may be of glass or plastic, for example. The glass surface may for example, chemically strengthened glass. Chemically strengthened glass may be for example, alkali-alumino silicate glass. The glass surface may be laminated. This enables thin, light and damage-resistant surface for the apparatus.

The first touch screen 12 and the second touch screen 14 may be for example, 5-wire resistive, surface capacitive, projected capacitive, surface acoustic wave (SAW) or infrared touch screen. The touch screen may comprise four layers: a top polyester-coated layer with a transparent metallic-conductive coating on the bottom, an adhesive spacer, a glass layer coated with a transparent metallic-conductive coating on the top, and an adhesive layer on the backside of the glass for mounting.

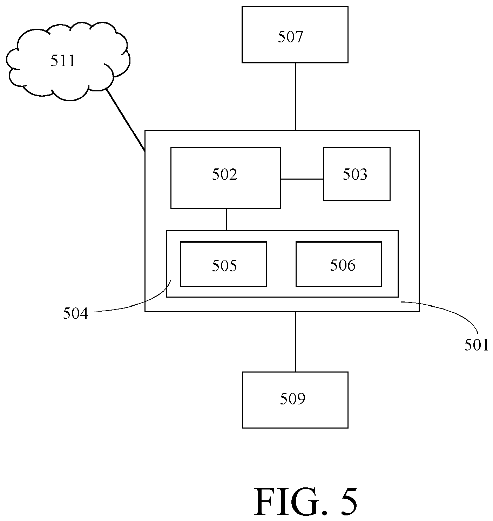

FIG. 5 illustrates a schematic diagram of an apparatus 500 that may be included in or configured to control as a dental care unit instrument bridge apparatus 10 according to some embodiments. The apparatus 500 comprises a computing unit 501 and may comprise or be connected to further units, such as one or more input devices 509. The apparatus may be connected to (further), other locally or remotely connectable devices 507, and network(s)/service(s) 511, such as a cloud service.

The computing unit 501 may comprise at least one processor 502, a communications unit 503 and a memory 504. The communication unit 503 may comprise at least one transmitter and receiver, which may be configured to operate in accordance with wired or wireless communication standard, such as global system for mobile communication, GSM, wideband code division multiple access, WCDMA, long term evolution, LTE, 5G or other cellular communications systems, wireless local area network, WLAN, wireless short range communication system, and/or Ethernet standards, for example.

The memory 504 may store computer program code 505 and parameters 506 for causing the computing unit to perform at least some of the presently disclosed features and features illustrated in FIG. 2 and further embodiments thereof, when the computer program code is executed by the processor 502.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

The verbs "to comprise" and "to include" are used in this document as open limitations that neither exclude nor require the existence of also un-recited features.

Furthermore, it is to be understood that the use of "a" or "an", that is, a singular form, throughout this document does not exclude a plurality.

## Claims

1. A dental care apparatus, comprising an instrument bridge (10) comprising a first touch screen (12), a second touch screen (14), and an instrument holder (16), wherein the first touch screen and the second touch screen are independently configurable, and the apparatus comprises means for causing the apparatus to:
- receive (200) a first input associated with a first operator accessing the apparatus, indicative at least of a role and/or dental treatment procedure for the first operator,
- receive (210) a second input associated with a second operator accessing the apparatus, indicative at least of a role and/or dental treatment procedure for the second operator,
- define (220) a first operator configuration on the basis of the received first input, wherein the first operator configuration is indicative of a first set of dental care unit control actions associated with the role and/or dental treatment procedure for the first operator,
- define (230) a second operator configuration on the basis of the received second input, wherein the second operator configuration is indicative of a second set of dental care unit control actions associated with the role and/or dental treatment procedure for the second operator,
- generate (240) a first display view for the first touch screen on the basis of the first operator configuration, wherein the first display view is specific for the role and/or dental treatment procedure for the first operator, and
- generate (250) a second display view for the second touch screen on the basis of the second operator configuration, wherein the second display view is specific for the role and/or dental treatment procedure for the second operator.

2. The apparatus of claim 1, wherein the first touch screen (12) is positioned at a first end of the apparatus, the second touch screen (14) is positioned at a second end of the apparatus, and the instrument holder is between the first touch screen and the second touch screen.

3. The apparatus of any preceding claim, wherein a one-piece transparent surface portion covers or comprises at least the first touch screen and the second touch screen, and the instrument holder (16) is attached to and/or positioned on top of the surface portion.

4. The apparatus of any preceding claim, wherein the means are configured to
- control a first set of dental care unit actuators based on the first set of dental care unit control actions, and
- control a second set of dental care unit actuators based on the second set of dental care unit control actions.

5. The apparatus of any preceding claim, wherein the first input comprises a first treatment identifier and the first operator configuration is defined by selecting a preconfigured treatment program associated with the first treatment identifier, the preconfigured treatment program comprising the first set of dental care unit control actions.

6. The apparatus of any preceding claim, wherein the first input and/or the second input is received in at least one push message from a cloud server, wherein the at least one push message comprises the first operator configuration and/or the second operator configuration, or a sequence of control parameters for the first operator configuration and/or the second operator configuration.

7. The apparatus of any preceding claim, wherein the first display view and/or the second display view comprises a dental treatment procedure view comprising a treatment stage specific display element configuration, selected among a set of treatment stage specific display element configurations for the dental treatment procedure based on a third input of current treatment stage, and the means are configured to detect (300) a fourth input to change treatment stage and update (320) the dental treatment procedure view in the first display view and/or the second display view in response to the fourth input.

8. The apparatus of any preceding claim, wherein the means are configured to select one or more operator assistance display elements in the first display view and/or the second display view in accordance with the first operator configuration and/or the second operator configuration.

9. The apparatus of any preceding claim, wherein the means are configured to identify a dental instrument detached from the instrument holder (16), and in response to identifying the dental instrument, control the first touch screen and/or the second touch screen to change the current view and display information associated with the identified dental instrument.

10. A method for controlling a dental care apparatus comprising an instrument bridge (10) comprising a first touch screen (12), a second touch screen (14), and an instrument holder (16), wherein the first touch screen and the second touch screen are independently configurable, the method comprising:
- receiving (200) a first input associated with a first operator accessing the apparatus, indicative at least of a role and/or dental treatment procedure for the first operator,
- receiving (210) a second input associated with a second operator accessing the apparatus, indicative at least of a role and/or dental treatment procedure for the second operator,
- defining (220) a first operator configuration on the basis of the received first input, wherein the first operator configuration is indicative of a first set of dental care unit control actions associated with the role and/or dental treatment procedure for the first operator,
- defining (230) a second operator configuration on the basis of the received second input, wherein the second operator configuration is indicative of a second set of dental care unit control actions associated with the role and/or dental treatment procedure for the second operator,
- generating (240) a first display view for the first touch screen on the basis of the first operator configuration, wherein the first display view is specific for the role and/or dental treatment procedure for the first operator, and
- generating (250) a second display view for the second touch screen on the basis of the second operator configuration, wherein the second display view is specific for the role and/or dental treatment procedure for the second operator.

11. The method of claim 10, comprising:
- control a first set of dental care unit actuators based on the first set of dental care unit control actions, and
- control a second set of dental care unit actuators based on the second set of dental care unit control actions.

12. The method of claim 10 or 11, wherein the first display view and/or the second display view comprises a dental treatment procedure view comprising a treatment stage specific display element configuration, selected among a set of treatment stage specific display element configurations for the dental treatment procedure based on a third input of current treatment stage, the method comprising: detecting (300) a fourth input to change treatment stage and updating (320) the dental treatment procedure view in the first display view and/or the second display view in response to the fourth input.

13. The method of any preceding claim 10 to 13, comprising identifying a dental instrument detached from the instrument holder section (16), and controlling the first touch screen and/or the second touch screen to change the current view and display information associated with the identified dental instrument.

14. A computer program, comprising code configured, when executed in a data processing apparatus, to carry out the method of any preceding claim 10 to 13.

15. A dental care unit, comprising an accessory portion, a patient chair, and the apparatus of any preceding claim 1-9.

## Patentansprüche

1. Zahnpflegeeinrichtung, die eine Instrumentenbrücke (10) umfasst, die einen ersten Touchscreen (12), einen zweiten Touchscreen (14) und einen Instrumentenhalter (16) umfasst, wobei der erste Touchscreen und der zweite Touchscreen unabhängig voneinander konfigurierbar sind und die Einrichtung Mittel umfasst, um die Einrichtung zu veranlassen:
- eine erste Eingabe zu empfangen (200), die mit einem ersten Bediener, der auf die Einrichtung zugreift, verknüpft ist, die auf mindestens eine Rolle und/oder einen Zahnbehandlungsvorgang für den ersten Bediener hinweist,
- eine zweite Eingabe zu empfangen (210), die mit einem zweiten Bediener, der auf die Einrichtung zugreift, verknüpft ist, die auf mindestens eine Rolle und/oder einen Zahnbehandlungsvorgang für den zweiten Bediener hinweist,
- eine erste Bedienerkonfiguration auf Basis der empfangenen ersten Eingabe zu definieren (220), wobei die erste Bedienerkonfiguration auf einen ersten Satz an Steueraktionen der Zahnpflegeeinheit, die mit der Rolle und/oder dem Zahnbehandlungsvorgang verknüpft sind, für den ersten Bediener hinweist,
- eine zweite Bedienerkonfiguration auf Basis der empfangenen zweiten Eingabe zu definieren (230), wobei die zweite Bedienerkonfiguration auf einen zweiten Satz an Steueraktionen der Zahnpflegeeinheit, die mit der Rolle und/oder dem Zahnbehandlungsvorgang verknüpft sind, für den zweiten Bediener hinweist,
- eine erste Anzeigeansicht für den ersten Touchscreen auf Basis der ersten Bedienerkonfiguration zu erzeugen (240), wobei die erste Anzeigeansicht für die Rolle und/oder den Zahnbehandlungsvorgang für den ersten Bediener spezifisch ist, und
- eine zweite Anzeigeansicht für den zweiten Touchscreen auf Basis der zweiten Bedienerkonfiguration zu erzeugen (250), wobei die zweite Anzeigeansicht für die Rolle und/oder den Zahnbehandlungsvorgang für den zweiten Bediener spezifisch ist.

2. Einrichtung nach Anspruch 1, wobei der erste Touchscreen (12) an einem ersten Ende der Einrichtung positioniert ist, der zweite Touchscreen (14) an einem zweiten Ende der Einrichtung positioniert ist, und der Instrumentenhalter sich zwischen dem ersten Touchscreen und dem zweiten Touchscreen befindet.

3. Einrichtung nach einem vorstehenden Anspruch, wobei ein einteiliger transparenter Oberflächenabschnitt mindestens den ersten Touchscreen und den zweiten Touchscreen bedeckt oder umfasst, und der Instrumentenhalter (16) oben auf dem Oberflächenabschnitt befestigt und/oder positioniert ist.

4. Einrichtung nach einem vorstehenden Anspruch, wobei die Mittel konfiguriert sind, um
- einen ersten Satz an Betätigungsvorrichtungen der Zahnpflegeeinheit basierend auf dem ersten Satz an Steueraktionen der Zahnpflegeeinheit zu steuern, und
- einen zweiten Satz an Betätigungsvorrichtungen der Zahnpflegeeinheit basierend auf dem zweiten Satz an Steueraktionen der Zahnpflegeeinheit zu steuern.

5. Einrichtung nach einem vorstehenden Anspruch, wobei die erste Eingabe eine erste Behandlungskennung umfasst und die erste Bedienerkonfiguration durch Auswählen eines vorkonfigurierten Behandlungsprogramms, das mit der ersten Behandlungskennung verknüpft ist, definiert wird, wobei das vorkonfigurierte Behandlungsprogramm den ersten Satz an Steueraktionen der Zahnpflegeeinheit umfasst.

6. Einrichtung nach einem vorstehenden Anspruch, wobei die erste Eingabe und/oder die zweite Eingabe in mindestens einer Push-Nachricht von einem Cloud-Server empfangen wird, wobei die mindestens eine Push-Nachricht die erste Bedienerkonfiguration und/oder die zweite Bedienerkonfiguration oder eine Abfolge von Steuerparametern für die erste Bedienerkonfiguration und/oder die zweite Bedienerkonfiguration umfasst.

7. Einrichtung nach einem vorstehenden Anspruch, wobei die erste Anzeigeansicht und/oder die zweite Anzeigeansicht eine Ansicht des Zahnbehandlungsvorgangs umfasst, die eine behandlungsstufenspezifische Anzeigeelementkonfiguration, ausgewählt aus einem Satz an behandlungsstufenspezifischen Anzeigeelementkonfigurationen für den Zahnbehandlungsvorgang basierend auf einer dritten Eingabe der aktuellen Behandlungsstufe, umfasst, und die Mittel konfiguriert sind, um eine vierte Eingabe zu erkennen (300), um die Behandlungsstufe zu ändern und als Reaktion auf die vierte Eingabe die Ansicht des Zahnbehandlungsvorgangs in der ersten Anzeigeansicht und/oder der zweiten Anzeigeansicht zu aktualisieren (320).

8. Einrichtung nach einem vorstehenden Anspruch, wobei die Mittel konfiguriert sind, um ein oder mehrere Bediener-Hilfsanzeigeelemente in der ersten Anzeigeansicht und/oder der zweiten Anzeigeansicht in Übereinstimmung mit der ersten Bedienerkonfiguration und/oder der zweiten Bedienerkonfiguration auszuwählen.

9. Einrichtung nach einem vorstehenden Anspruch, wobei die Mittel konfiguriert sind, ein aus dem Instrumentenhalter (16) herausgenommenes zahnärztliches Instrument zu identifizieren und als Reaktion auf das Identifizieren des zahnärztlichen Instruments den ersten Touchscreen und/oder den zweiten Touchscreen zu steuern, um die aktuelle Ansicht zu ändern und Informationen, die mit dem identifizierten zahnärztlichen Instrument verknüpft sind, anzuzeigen.

10. Verfahren zum Steuern einer Zahnpflegeeinrichtung, die eine Instrumentenbrücke (10) umfasst, die einen ersten Touchscreen (12), einen zweiten Touchscreen (14) und einen Instrumentenhalter (16) umfasst, wobei der erste Touchscreen und der zweite Touchscreen unabhängig voneinander konfigurierbar sind, wobei das Verfahren umfasst:
- Empfangen (200) einer ersten Eingabe, die mit einem ersten Bediener, der auf die Einrichtung zugreift, verknüpft ist, die auf mindestens eine Rolle und/oder einen Zahnbehandlungsvorgang für den ersten Bediener hinweist,
- Empfangen (210) einer zweiten Eingabe, die mit einem zweiten Bediener, der auf die Einrichtung zugreift, verknüpft ist, die auf mindestens eine Rolle und/oder einen Zahnbehandlungsvorgang für den zweiten Bediener hinweist,
- Definieren (220) einer ersten Bedienerkonfiguration auf Basis der empfangenen ersten Eingabe, wobei die erste Bedienerkonfiguration auf einen ersten Satz an Steueraktionen der Zahnpflegeeinheit, die mit der Rolle und/oder dem Zahnbehandlungsvorgang verknüpft sind, für den ersten Bediener hinweist,
- Definieren (230) einer zweiten Bedienerkonfiguration auf Basis der empfangenen zweiten Eingabe, wobei die zweite Bedienerkonfiguration auf einen zweiten Satz an Steueraktionen der Zahnpflegeeinheit, die mit der Rolle und/oder dem Zahnbehandlungsvorgang verknüpft sind, für den zweiten Bediener hinweist,
- Erzeugen (240) einer ersten Anzeigeansicht für den ersten Touchscreen auf Basis der ersten Bedienerkonfiguration, wobei die erste Anzeigeansicht für die Rolle und/oder den Zahnbehandlungsvorgang für den ersten Bediener spezifisch ist, und
- Erzeugen (250) einer zweiten Anzeigeansicht für den zweiten Touchscreen auf Basis der zweiten Bedienerkonfiguration, wobei die zweite Anzeigeansicht für die Rolle und/oder den Zahnbehandlungsvorgang für den zweiten Bediener spezifisch ist.

11. Verfahren nach Anspruch 10, umfassend:
- einen ersten Satz an Betätigungsvorrichtungen der Zahnpflegeeinheit basierend auf dem ersten Satz an Steueraktionen der Zahnpflegeeinheit zu steuern, und
- einen zweiten Satz an Betätigungsvorrichtungen der Zahnpflegeeinheit basierend auf dem zweiten Satz an Steueraktionen der Zahnpflegeeinheit zu steuern.

12. Verfahren nach Anspruch 10 oder 11, wobei die erste Anzeigeansicht und/oder die zweite Anzeigeansicht eine Ansicht des Zahnbehandlungsvorgangs umfasst, die eine behandlungsstufenspezifische Anzeigeelementkonfiguration, ausgewählt aus einem Satz an behandlungsstufenspezifischen Anzeigeelementkonfigurationen für den Zahnbehandlungsvorgang basierend auf einer dritten Eingabe der aktuellen Behandlungsstufe, umfasst, wobei das Verfahren umfasst: Erkennen (300) einer vierten Eingabe, um die Behandlungsstufe zu ändern und Aktualisieren (320) Ansicht des Zahnbehandlungsvorgangs in der ersten Anzeigeansicht und/oder der zweiten Anzeigeansicht als Reaktion auf die vierte Eingabe.

13. Verfahren nach einem vorstehenden Anspruch 10 bis 13, das Identifizieren eines aus dem Instrumentenhalterabschnitt (16) herausgenommenen zahnärztlichen Instruments und Steuern des ersten Touchscreens und/oder des zweiten Touchscreens umfasst, um die aktuelle Ansicht zu ändern und Informationen, die mit dem identifizierten zahnärztlichen Instrument verknüpft sind, anzuzeigen.

14. Computerprogramm, das Code umfasst, der konfiguriert ist, um, wenn er in einer Datenverarbeitungseinrichtung ausgeführt wird, das Verfahren nach einem vorstehenden Anspruch 10 bis 13 auszuführen.

15. Zahnpflegeeinheit, die einen Zubehörabschnitt, einen Patientenstuhl und die Einrichtung nach einem vorstehenden Anspruch 1-9 umfasst.

## Revendications

1. Appareil de soins dentaires, comprenant un pont d'instrument (10) comprenant un premier écran tactile (12), un second écran tactile (14) et un support d'instrument (16), dans lequel le premier écran tactile et le second écran tactile sont configurables indépendamment, et l'appareil comprend des moyens pour amener l'appareil à :
- recevoir (200) une première entrée associée à un premier opérateur accédant à l'appareil, indicative au moins d'un rôle et/ou d'une procédure de traitement dentaire pour le premier opérateur,
- recevoir (210) une deuxième entrée associée à un second opérateur accédant à l'appareil, indicative au moins d'un rôle et/ou d'une procédure de traitement dentaire pour le second opérateur,
- définir (220) une première configuration d'opérateur sur la base de la première entrée reçue, dans lequel la première configuration d'opérateur est indicative d'un premier ensemble d'actions de commande d'unité de soins dentaires associé au rôle et/ou à la procédure de traitement dentaire pour le premier opérateur,
- définir (230) une seconde configuration d'opérateur sur la base de la deuxième entrée reçue, dans lequel la seconde configuration d'opérateur est indicative d'un second ensemble d'actions de commande d'unité de soins dentaires associé au rôle et/ou à la procédure de traitement dentaire pour le second opérateur,
- générer (240) une première vue d'affichage pour le premier écran tactile sur la base de la première configuration d'opérateur, dans lequel la première vue d'affichage est spécifique au rôle et/ou à la procédure de traitement dentaire pour le premier opérateur, et
- générer (250) une seconde vue d'affichage pour le second écran tactile sur la base de la seconde configuration d'opérateur, dans lequel la seconde vue d'affichage est spécifique au rôle et/ou à la procédure de traitement dentaire pour le second opérateur.

2. Appareil selon la revendication 1, dans lequel le premier écran tactile (12) est positionné à une première extrémité de l'appareil, le second écran tactile (14) est positionné à une seconde extrémité de l'appareil, et le support d'instrument se trouve entre le premier écran tactile et le second écran tactile.

3. Appareil selon une quelconque revendication précédente, dans lequel une partie de surface transparente d'un seul tenant couvre ou comprend au moins le premier écran tactile et le second écran tactile, et le support d'instrument (16) est attaché à et/ou positionné sur le dessus de la partie de surface.

4. Appareil selon une quelconque revendication précédente, dans lequel les moyens sont configurés pour
- commander un premier ensemble d'actionneurs d'unité de soins dentaires sur la base du premier ensemble d'actions de commande d'unité de soins dentaires, et
- commander un second ensemble d'actionneurs d'unité de soins dentaires sur la base du second ensemble d'actions de commande d'unité de soins dentaires.

5. Appareil selon une quelconque revendication précédente, dans lequel la première entrée comprend un premier identifiant de traitement et la première configuration d'opérateur est définie en sélectionnant un programme de traitement préconfiguré associé au premier identifiant de traitement, le programme de traitement préconfiguré comprenant le premier ensemble d'actions de commande d'unité de soins dentaires.

6. Appareil selon une quelconque revendication précédente, dans lequel la première entrée et/ou la deuxième entrée est reçue dans au moins un message push provenant d'un serveur en nuage, dans lequel le au moins un message push comprend la première configuration d'opérateur et/ou la seconde configuration d'opérateur, ou une séquence de paramètres de commande pour la première configuration d'opérateur et/ou la seconde configuration d'opérateur.

7. Appareil selon une quelconque revendication précédente, dans lequel la première vue d'affichage et/ou la seconde vue d'affichage comprend une vue de procédure de traitement dentaire comprenant une configuration d'élément d'affichage spécifique à l'étape de traitement, sélectionnée parmi un ensemble de configurations d'élément d'affichage spécifiques à l'étape de traitement pour la procédure de traitement dentaire sur la base d'une troisième entrée de l'étape de traitement actuelle, et les moyens sont configurés pour détecter (300) une quatrième entrée pour changer d'étape de traitement et mettre à jour (320) la vue de procédure de traitement dentaire dans la première vue d'affichage et/ou la seconde vue d'affichage en réponse à la quatrième entrée.

8. Appareil selon une quelconque revendication précédente, dans lequel les moyens sont configurés pour sélectionner un ou plusieurs éléments d'affichage d'assistance à l'opérateur dans la première vue d'affichage et/ou la seconde vue d'affichage conformément à la première configuration d'opérateur et/ou la seconde configuration d'opérateur.

9. Appareil selon une quelconque revendication précédente, dans lequel les moyens sont configurés pour identifier un instrument dentaire détaché du support d'instrument (16) et, en réponse à l'identification de l'instrument dentaire, commander le premier écran tactile et/ou le second écran tactile pour changer la vue actuelle et afficher des informations associées à l'instrument dentaire identifié.

10. Procédé de commande d'un appareil de soins dentaires comprenant un pont d'instrument (10) comprenant un premier écran tactile (12), un second écran tactile (14) et un support d'instrument (16), dans lequel le premier écran tactile et le second écran tactile sont configurables indépendamment, le procédé comprenant les étapes consistant à :
- recevoir (200) une première entrée associée à un premier opérateur accédant à l'appareil, indicative au moins d'un rôle et/ou d'une procédure de traitement dentaire pour le premier opérateur,
- recevoir (210) une deuxième entrée associée à un second opérateur accédant à l'appareil, indicative au moins d'un rôle et/ou d'une procédure de traitement dentaire pour le second opérateur,
- définir (220) une première configuration d'opérateur sur la base de la première entrée reçue, dans lequel la première configuration d'opérateur est indicative d'un premier ensemble d'actions de commande d'unité de soins dentaires associé au rôle et/ou à la procédure de traitement dentaire pour le premier opérateur,
- définir (230) une seconde configuration d'opérateur sur la base de la deuxième entrée reçue, dans lequel la seconde configuration d'opérateur est indicative d'un second ensemble d'actions de commande d'unité de soins dentaires associé au rôle et/ou à la procédure de traitement dentaire pour le second opérateur,
- générer (240) une première vue d'affichage pour le premier écran tactile sur la base de la première configuration d'opérateur, dans lequel la première vue d'affichage est spécifique au rôle et/ou à la procédure de traitement dentaire pour le premier opérateur, et
- générer (250) une seconde vue d'affichage pour le second écran tactile sur la base de la seconde configuration d'opérateur, dans lequel la seconde vue d'affichage est spécifique au rôle et/ou à la procédure de traitement dentaire pour le second opérateur.

11. Procédé selon la revendication 10, comprenant les étapes consistant à :
- commander un premier ensemble d'actionneurs d'unité de soins dentaires sur la base du premier ensemble d'actions de commande d'unité de soins dentaires, et
- commander un second ensemble d'actionneurs d'unité de soins dentaires sur la base du second ensemble d'actions de commande d'unité de soins dentaires.

12. Procédé selon la revendication 10 ou 11, dans lequel la première vue d'affichage et/ou la seconde vue d'affichage comprend une vue de procédure de traitement dentaire comprenant une configuration d'élément d'affichage spécifique à l'étape de traitement, sélectionnée parmi un ensemble de configurations d'élément d'affichage spécifiques à l'étape de traitement pour la procédure de traitement dentaire sur la base d'une troisième entrée de l'étape de traitement actuelle, le procédé comprenant les étapes consistant à : détecter (300) une quatrième entrée pour changer d'étape de traitement et mettre à jour (320) la vue de procédure de traitement dentaire dans la première vue d'affichage et/ou la seconde vue d'affichage en réponse à la quatrième entrée.

13. Procédé selon une quelconque revendication 10 à 13 précédente, comprenant l'identification d'un instrument dentaire détaché de la section de support d'instrument (16) et la commande du premier écran tactile et/ou du second écran tactile pour changer la vue actuelle et afficher des informations associées à l'instrument dentaire identifié.

14. Programme informatique, comprenant un code configuré pour, lorsqu'il est exécuté dans un appareil de traitement de données, mettre en œuvre le procédé selon une quelconque revendication 10 à 13 précédente.

15. Unité de soins dentaires, comprenant une partie d'accessoire, une chaise de patient et l'appareil selon une quelconque revendication 1-9 précédente.
